# EUROPEAN PATENT SPECIFICATION

(11) **EP 4 342 425 B1**
(45) Date of publication and mention of the grant of the patent: **09.04.2025**
(21) Application number: 23192960.5
(22) Date of filing: 23.08.2023
(51) Int. Cl.: A61F 2/24

(54) **PROSTHETIC TRICUSPID HEART VALVE**
PROTHETISCHE TRIKUSPIDALHERZKLAPPE
VALVULE CARDIAQUE PROTHETIQUE TRICUSPIDE

(30) Priority: 21.09.2022 US 202263376493 P
(43) Date of publication of application: 27.03.2024
(73) Proprietor: St. Jude Medical, Cardiology Division, Inc., St. Paul MN 55117 (US)
(72) Inventor: King, Alec, Maple Grove, 6390 (US); Panus, David A., Maple Grove, 6329 (US); Peckels, William H., Robbinsdale, 55422 (US); Robinson, Paul, Minneapolis, 55410 (US); Marnach, Heath, Minneapolis, 55419 (US); Huddleston, Preston James, Maplewood, 55117 (US); Mai, Son T., North Branch, 55056 (US)
(74) Representative: LKGLOBAL Lorenz & Kopf Patentanwalt Attorney at Law PartG mbB

(56) References cited:
- WO-A1-2013/059747
- FR-A1- 3 043 907
- US-A1- 2013 338 766
- US-A1- 2014 194 983

## Description

### Cross-Reference to Related Applications

This application claims priority to U.S. Provisional Patent Application No. 63/376,493, filed September 21, 2022.

### Background of the Disclosure

The heart has four native valves, including the aortic valve, the pulmonary valve, the mitral valve (also known as the left atrioventricular valve), and the tricuspid valve (also known as the right atrioventricular valve). When these valves begin to fail, for example by not fully coapting and allowing retrograde blood flow (or regurgitation) across the valve, it may be desirable to repair or replace the valve. Prosthetic replacement heart valves may be surgically implanted via an open chest, open-heart procedure while the patient is on cardiopulmonary bypass. However, such procedures are extremely invasive, and frail patients, who may be the most likely to need a prosthetic heart valve, may not be likely to survive such a procedure. Prosthetic heart valves have been trending toward less invasive procedures, including collapsible and expandable heart valves that can be delivered through the vasculature in a transcatheter procedure.

The aortic and pulmonary valves typically have a relatively circular shape and a relatively small diameter compared to the left and right atrioventricular valves. As a result, transcatheter prosthetic heart valves designed for the mitral and tricuspid valve may have more significant challenges that need to be overcome compared to transcatheter prosthetic heart valve designs for the aortic and pulmonary valves.

FR 3 043 907 A1 describes an assembly for the tricuspid orifice of a human heart, comprises an external frame connected to an internal stent carrying a tricuspid valve bioprosthesis and a sealing skirt. The external frame is configured to hold its position in the native tricuspid annulus. The internal stent is connected to the external frame by one or more fixing strands. The sealing skirt covers the interstitial space existing between the external frame and the internal stent. Developments are described which comprise in particular the use of a deformable region of the frame, various alternative embodiments of the sealing skirt, the use of a frame composed of multiple sub-sections, the use of fixing strands between the stent and the frame and of fixing elements for fixing the assembly to the native tissue, the use of sensors and/or actuators, and also the use of a stent in the inferior vena cava.

### Brief Summary

One or more of the above-mentioned problems and challenges are addressed by the subject-matter of claim 1. Further embodiment in accordance with the invention are described in the dependent claims.

According to one aspect of the disclosure, a prosthetic heart valve includes a collapsible and expandable frame that, in an expanded condition, includes a central portion, an atrial portion flaring radially outwardly from the central portion, and a ventricular portion flaring radially outwardly from the central portion. A tube is positioned within the frame, the tube having a lumen extending along a longitudinal axis from the atrial portion toward the ventricular portion of the frame, wherein the tube is formed of tissue or fabric. A plurality of prosthetic leaflets are directly coupled to the tube to form a valve, the valve allowing blood to flow through the lumen of the tube in an antegrade direction but substantially blocking blood from flowing through the lumen of the tube in a retrograde direction. A plurality of cords each have a first end coupled to the frame and a second end coupled to the tube. Each of the plurality of cords extends in a radial direction toward the longitudinal axis. The tube excludes any metal structure directly attached to the tube. Each of the plurality of cords may be a suture. A skirt may be coupled to the frame, the skirt including an atrial portion extending radially inwardly from the frame and being connected to a first end of the tube, and a ventricular portion extending radially inwardly from the frame and being connected to a second end of the tube.

According to another aspect of the disclosure, a prosthetic heart valve includes a collapsible and expandable frame that, in an expanded condition, includes a central portion, an atrial portion flaring radially outwardly from the central portion, and a ventricular portion flaring radially outwardly from the central portion. A tube may be positioned within the frame, the tube having a lumen extending along a longitudinal axis from an inflow end to an outflow end, wherein the tube is formed of tissue or fabric. A plurality of first cords may each have a first end coupled to the frame and a second end coupled to the inflow end of the tube, the first plurality of cords maintaining the inflow end of the tube in an open condition. A pair of second cords may each have a first end coupled to the frame and a second end coupled to the outflow end of the tube, the pair of second cords coupled to diametrically opposed portions of the outflow end of the tube so that two free edges of the outflow end of the tube are capable of collapsing toward each other and opening away from each other. The plurality of first cords may be sutures, and the pair of second cords may be sutures. The tube may be formed of tissue that is rolled into a generally cylindrical shape. The tube may be formed as two pieces of fabric that are coupled together, via a pair of seams, to form a generally cylindrical shape, the pair of seams aligning with the pair of second cords. The prosthetic heart valve may exclude prosthetic leaflets separate from the tube.

According to a further aspect of the disclosure, a method of replacing an atrioventricular heart valve of a heart may include expanding a frame into the heart valve, the frame including a central portion in contact with an annulus of the heart valve, an atrial portion flaring radially outwardly from the central portion, and a ventricular portion flaring radially outwardly from the central portion. A tube may be suspended within the frame, the tube being suspended by a plurality of cords each having a first end coupled to the frame and a second end coupled to the tube, the tube having a lumen extending along a longitudinal axis from an inflow end to an outflow end, the tube being formed of tissue or fabric, each of the plurality of sutures extending in a radial direction toward the longitudinal axis. After expanding the frame into the heart valve, blood may flow in an antegrade direction from an atrium to a ventricle through the tube during atrial systole, but blood may be prevented from flowing in a retrograde direction from the ventricle to the atrium through the tube during ventricular systole. The tube may move toward the atrium and then toward the ventricle while the heart cycles between atrial systole and ventricular systole, but the frame may remain stationary as the heart cycles between atrial systole and ventricular systole. A plurality of prosthetic leaflets may be directly coupled to the tube to form a valve. The plurality of cords may include a plurality of first cords each having a first end coupled to the frame and a second end coupled to the inflow end of the tube, the first plurality of cords maintaining the inflow end of the tube in an open condition. The plurality of cords may include a pair of second cords each having a first end coupled to the frame and a second end coupled to the outflow end of the tube, the pair of second cords coupled to diametrically opposed portions of the outflow end of the tube so that two free edges of the outflow end of the tube are capable of collapsing toward each other and opening away from each other. The tube may be formed of tissue that is rolled into a generally cylindrical shape. The tube may be formed as two pieces of fabric that are coupled together, via a pair of seams, to form a generally cylindrical shape, the pair of seams aligning with the pair of second cords.

According to still another aspect of the disclosure, a method of replacing a right atrioventricular valve of a heart of a patient may include delivering an anchor to a superior vena cava of the patient. The anchor may be expanded into the superior vena cava. After expanding the anchor, a prosthetic heart valve may be delivered to the right atrioventricular valve. The prosthetic heart valve may be expanded within the right atrioventricular valve while a tether is coupled to the prosthetic heart valve. The tether may be tensioned and fixed to the anchor while the tether is tensioned. Fixing the tether may be performed after expanding the anchor and after expanding the prosthetic heart valve. Expanding the prosthetic heart valve may include positioning a pair of projections in contact with tissue of the right atrioventricular valve on an outflow side of the right atrioventricular valve. After expanding the prosthetic heart valve within the right atrioventricular valve, the prosthetic heart valve may not be in contact with tissue of the right atrioventricular valve on an inflow side of the right atrioventricular valve. Tensioning the tether may be performed by pulling the tether proximally while the tether is looped over an arch of the anchor. Tensioning the tether may be performed by pulling the tether proximally while the tether is extending through a tether connection mechanism of the anchor, and fixing the tether may be performed by releasing force on the tether whereby a tine or barb of the anchor penetrates the tether to maintain the tether in a tensioned state.

### Brief Description of the Drawings

Fig. 1 is a schematic illustration of the right atrioventricular valve.
Fig. 2 is a perspective view of a prosthetic heart valve according to an embodiment of the disclosure, as viewed from the outflow end of the prosthesis.
Fig. 3 is a perspective view of the prosthetic heart valve of Fig. 2 prior to prosthetic leaflets being attached thereto.
Fig. 4 is a side view of an exemplary frame for use with the prosthetic heart valve of Fig. 2, the frame being shown in an expanded condition.
Fig. 5 is a highly schematic view of an alternate version of the prosthetic heart valve of Fig. 2.
Figs. 6A-B show the prosthetic heart valve of Fig. 2 in a testing device simulating flow during atrial systole and ventricular systole, respectively.
Fig. 7A is a cutaway view of the heart showing a schematic of a prosthetic tricuspid valve system implanted in the native tricuspid valve.
Fig. 7B is a highly schematic view of the prosthetic heart valve of the system of Fig. 7A.
Fig. 7C is a top view of the prosthetic heart valve of Fig. 7B, as viewed from the inflow-to-outflow direction.
Fig. 7D is a schematic cutaway view of the anchor of the tricuspid valve system of Fig. 7A deployed into a superior vena cava.
Fig. 7E shows the anchor of Fig. 7D isolated from other components of the tricuspid valve system of Fig. 7A.
Figs. 7F-H illustrate different stages of the implantation of the prosthetic heart valve system of Fig. 7A.
Fig. 7I is a schematic view of an alternate version of the anchor of Fig. 7D.

### Detailed Description

As used herein, the term "inflow end," when used in connection with a prosthetic heart valve, refers to an end of the prosthetic heart valve into which blood first flows when the prosthetic heart valve is implanted in an intended position and orientation. On the other hand, the term "outflow end," when used in connection with a prosthetic heart valve, refers to the end of the prosthetic heart valve through which blood exits when the prosthetic heart valve is implanted in an intended position and orientation. In the figures, like numbers refer to like or identical parts. As used herein, the terms "substantially," "generally," "approximately," and "about" are intended to mean that slight deviations from absolute are included within the scope of the term so modified. When ranges of values are described herein, those ranges are intended to include sub-ranges. For example, a recited range of 1 to 10 includes 2, 5, 7, and other single values, as well as all sub-ranges within the range, such as 2 to 6, 3 to 9, 4 to 5, and others.

The present disclosure is generally directed to collapsible prosthetic tricuspid valves. Unless stated otherwise, the term "tricuspid valve" as used herein refers to the right atrioventricular valve, as opposed to being a generic term for a three-leaflet valve. Despite the above, it should be understood that the features described herein may apply to other types of prosthetic heart valves, including prosthetic heart valves that are adapted for use in other heart valves, such as the mitral heart valve. Further, the features of the prosthetic heart valves described herein may, in some circumstances, be suitable for surgical (e.g., non-collapsible) prosthetic heart valves. However, as noted above, the disclosure is provided herein in the context of a collapsible and expandable prosthetic tricuspid valve.

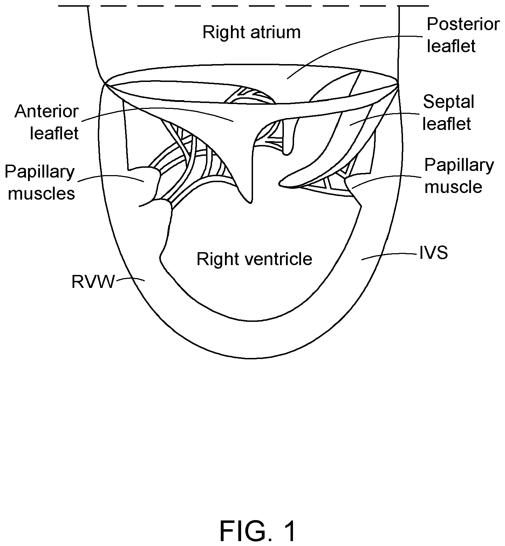

Fig. 1 is a schematic illustration of the right atrioventricular valve (commonly referred to as the tricuspid valve). The tricuspid valve separates the right atrium from the right ventricle, and typically includes three leaflets, which include a posterior leaflet, an anterior leaflet, and a septal leaflet. The septal leaflet is positioned nearest the interventricular septum ("IVS"). The tricuspid valve annulus may include conduction nodes near the connection point between the annulus and the septal leaflet, including for example the atrioventricular node ("AV node"). The AV node may typically be positioned on the atrial side of the native tricuspid valve annulus. Electrical impulses may be conducted from the AV node, via the bundle of His, to the Purkinje fibers that provide electrical conduction to the ventricles. Papillary muscles along the right ventricular wall ("RVW") may support chordae tendineae coupled to the tricuspid valve leaflets to prevent inversion of the leaflets during normal physiological operation. The left atrioventricular valve (commonly referred to as the mitral valve) may have a generally similar structure as the tricuspid valve, although many differences do exist - including for example the mitral valve typically includes two leaflets (an anterior and posterior leaflet) and has the general shape of a hyperbolic paraboloid or "saddle"-type shape. Both the mitral valve annulus and tricuspid valve annulus may be very large compared to the aortic and pulmonary valves. The tricuspid valve can range from between about 35 mm to about 65 mm in perimeter-derived diameter. However, it should be understood that these sizes are merely exemplary.

Because of the large sizes of the mitral valve and the tricuspid valve, it may be desirable for a collapsible prosthetic mitral or tricuspid valve to have a dual-frame design. In other words, a first large outer frame may be used primarily to anchor and/or seal the prosthetic heart valve at the native annulus, with a second smaller inner frame connected to and positioned within the outer frame. The inner frame may function primarily to support one or more prosthetic valve leaflets. In some instances, the inner frame may be generally cylindrical when implanted, with the inner and outer frames connected in a way such that forces from the native valve that deform the outer frame tend not to deform the inner frame (or at least not to a significant enough extent to reduce the ability of the prosthetic leaflets within the inner frame to properly coapt). Having a single large frame that serves both the anchoring/sealing function as well as directly supporting prosthetic leaflets may be undesirable in the tricuspid and mitral valves because the leaflets may need to be very large and may be more likely to be deformed during regular operation due to forces from the native annulus. Some embodiments described below provide the ability to have a single supporting frame for anchoring while avoiding at least some of the concerns described above for a single-frame mitral or tricuspid valve prosthesis. And while these embodiments may be suitable for either mitral or tricuspid valve replacement, they may be particularly suited for tricuspid valve replacement because of the lower forces and pressures that occur within and across the tricuspid valve compared to the mitral valve.

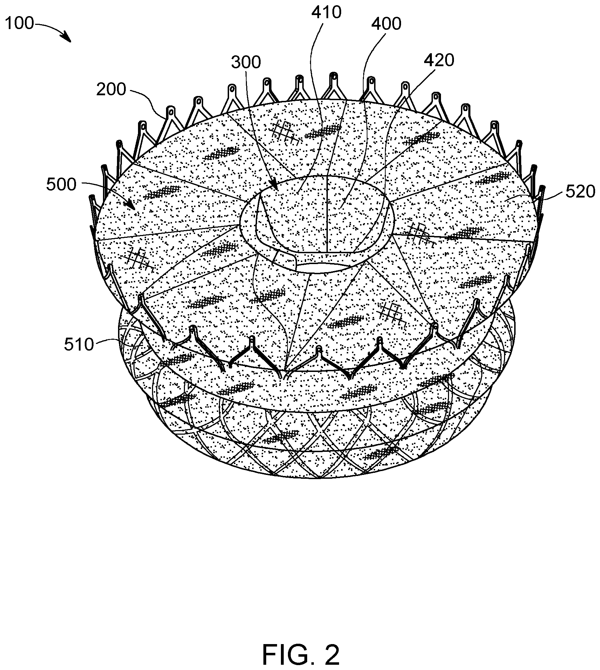

Fig. 2 shows a perspective view of a collapsible and expandable prosthetic heart valve 100. Although prosthetic heart valve 100 may be suitable as a replacement for a native mitral or tricuspid valve, prosthetic heart valve 100 is generally described below in the context of a prosthetic tricuspid valve. A main structural component of the prosthetic heart valve 100 is a frame 200. Portions of the frame 200 are covered by a liner or skirt material in Fig. 2.

Before returning to describe other portions of the prosthetic heart valve 100, an exemplary frame 200 is described. However, it should be understood that frame 200 is merely exemplary, and other frames having generally similar overall designs may be used in place of the frame 200 without a significant deviation from the functionality of the prosthetic heart valve 100.

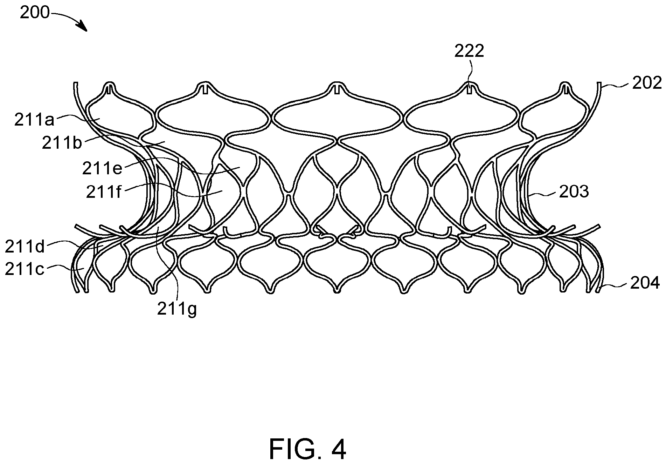

One exemplary option for the design of the frame 200 is shown in Fig. 4. Fig. 4 shows the frame 200 from a side view while in the expanded condition, with the inflow end of the frame 200 being positioned toward the top of Fig. 4 and the outflow end of the frame 200 being positioned toward the bottom of Fig. 4. Frame 200 may be formed of a superelastic and/or shape memory material such as Nitinol. According to some examples, other biocompatible metals or metal alloys may be suitable. For example, superelastic and/or self-expanding metals other than Nitinol may be suitable, while still other metals or metal alloys such as cobalt-chromium or stainless steel may be suitable, particularly if the stent or support structure is intended to be balloon expandable. In some examples, the frame 200 may be laser cut from a single tube, such as a shape memory metal tube. The shape memory metal tube may be Nitinol or any other bio-compatible metal tube. In some embodiments, the frame 200 may be formed of a shape memory polymer.

The frame 200 may be adapted to expand from a collapsed or constrained configuration to an expanded configuration. According to some examples, the frame 200 may be adapted to self-expand, although the frame could instead be partially or fully expandable by other mechanisms, such as balloon expansion. The frame 200 may be maintained in the collapsed configuration during delivery, for example via one or more overlying sheaths that restrict the frame from expanding. The frame 200 may be expanded during deployment from the delivery device once the delivery device is positioned within or adjacent to the native valve annulus. In the expanded configuration, an atrial portion 202 and ventricular portion 204 may extend radially outward from a central longitudinal axis of the frame 200 and/or a central portion 203 of the frame 200 and may be considered to flare outward relative to the central longitudinal axis of the frame 200 and/or central portion 203. The atrial portion 202 and ventricular portion 204 may be considered flanged relative to central portion 203. In some embodiments, the flared configuration of the atrial and ventricular portions 202, 204 and the central portion 203 may define a general hourglass shape in a side view of the frame 200. That is, the atrial and ventricular portions 202, 204 may be flared outwards relative to the central portion 203 and then curve or bend to point at least partially back in the axial direction. It should be understood, however, that an hourglass configuration is not limited to a symmetrical configuration. Atrial portion 202 may be referred to herein as an atrial portion, an atrial cuff, or an atrial anchor. Similarly, ventricular portion 204 may be referred to herein as a ventricular portion, a ventricular cuff, or a ventricular anchor. It should be understood that, in this context, the terms "portion," "cuff," and "anchor" are intended to be used interchangeably with each other.

As noted above, the frame 200 may include an atrial portion or anchor 202, a ventricular portion or anchor 204, and a central portion 203 coupling the atrial portion to the ventricular portion. The atrial portion and ventricular portion may be referred to herein as atrial or ventricular disks. Atrial portion 202 may be configured and adapted to be disposed on an atrial side of a native valve annulus and may flare radially outwardly from the central portion 203. Ventricular portion 204 may be configured and adapted to be disposed on a ventricular side of the native valve annulus and may also flare radially outwardly from the central portion 203. The central portion 203 may be configured to be situated in the valve orifice, for example in contact with the native valve annulus. In use, the atrial portion 202 and ventricular portion 204 effectively clamp the native valve annulus on the atrial and ventricular sides thereof, respectively, anchoring the prosthetic heart valve 100 in place.

The atrial portion 202 may be formed as a portion of a stent or other support structure that includes or is formed by a plurality of generally diamond-shaped cells, although other suitable cell shapes, such as triangular, quadrilateral, or polygonal may be appropriate. In some examples, the atrial portion 202 may be formed as a braided mesh, as a portion of a unitary stent, or a combination thereof. According to one example, the stent that includes the atrial portion 202 may be laser cut from a tube of Nitinol and heat set to the desired shape so that the stent, including atrial portion 202, is collapsible for delivery, and re-expandable to the set shape during deployment. The atrial portion 202 may be heat set into a suitable shape to conform to the native anatomy of the valve annulus to help provide a seal and/or anchoring between the atrial portion 202 and the native valve annulus. The shape-set atrial portion 202 may be partially or entirely covered by a cuff or skirt, on the luminal and/or abluminal surface of the atrial portion 202. The skirt may be formed of any suitable material, including biomaterials such as bovine pericardium, biocompatible polymers such as ultra-high molecular weight polyethylene ("UHMWPE"), woven polyethylene terephthalate ("PET") or expanded polytetrafluoroethylene ("ePTFE"), or combinations thereof. The atrial portion 202 may include features for connecting the atrial portion to a delivery system. For example, the atrial portion 202 may include pins or tabs 222 around which sutures (or suture loops) of the delivery system may wrap so that while the suture loops are wrapped around the pins or tabs 222, the frame 200 maintains a connection to the delivery device. However, it should be understood that pins or tabs 222 may be completely optional.

The ventricular portion 204 may also be formed as a portion of a stent or other support structure that includes or is formed of a plurality of diamond-shaped cells, although other suitable cell shapes, such as triangular, quadrilateral, or polygonal may be appropriate. In some examples, the ventricular portion 204 may be formed as a braided mesh, as a portion of a unitary stent, or a combination thereof. According to one example, the stent that includes the ventricular portion 204 may be laser cut from a tube of Nitinol and set to the desired shape *(e.g.,* via heat treating) so that the ventricular portion 204 is collapsible for delivery, and re-expandable to the set shape during deployment. The ventricular portion 204 may be partially or entirely covered by a cuff or skirt, on the luminal and/or abluminal surface of the ventricular portion 204. The skirt may be formed of any suitable material described above in connection with the skirt of atrial portion 202. It should be understood that the atrial portion 202 and ventricular portion 204 may be formed as portions of a single support structure, such as a single stent or braided mesh. However, in other embodiments, the atrial portion 202 and ventricular portion 204 may be formed separately and coupled with one another.

The frame 200 may be configured to expand circumferentially (and radially) and foreshorten axially as the prosthetic heart valve 100 expands from the collapsed delivery configuration to the expanded deployed configuration. The frame 200 may define a plurality of atrial cells 211a, 211b in two circumferential rows. For example, the first row of atrial cells 211a may be generally diamond shaped and positioned on the inflow end of the frame 200. The second row of atrial cells 211b may be positioned at least partially between adjacent atrial cells 211a in the first row, with the atrial cells 211b in the second row being positioned farther from the inflow end than the first row of atrial cells 211a. The frame 200 may include twelve atrial cells 211a in the first row each having a diamond shape, and twelve atrial cells 211b in the second row each having a skewed diamond shape. This skewed diamond shape, which is wider nearer the inflow (or top) end and narrower nearer the outflow (or bottom) end, may assist in transitioning from twelve cells per row on the atrial side of the stent to twenty-four cells per row on the ventricular side. However, it should be understood that the particular number, shape, and configuration of atrial cells may be different than the specific embodiment shown.

The frame 200 may include a plurality of ventricular cells 211c in a first row, and another plurality of ventricular cells 211d in a second row. The first row of ventricular cells 211c may be at the outflow end of the frame 200, and the second row of ventricular cells 211d may be positioned farther from the outflow end than, and adjacent to, the first row of ventricular cells 211c. In the illustrated embodiment the first and second rows of ventricular cells 211c, 211d are all generally diamond-shaped and have substantially the same or identical size, with twenty-four cells in the first row of ventricular cells 211c and twenty-four cells in the second row of ventricular cells 211d. However, it should be understood that the particular number, shape, and configuration of ventricular cells may be different than the specific embodiment shown.

Frame 200 is also illustrated as including three rows of center cells. A first row of center cells 211e may be positioned adjacent to the atrial end of the frame 200, each cell 211e being positioned between a pair of adjacent atrial cells 211b. Each center cell 211e may be substantially diamond-shaped, but it should be understood that adjacent center cells 211e do not directly touch one another. The first row of center cells 211e may include twelve center cells 211e, with the combination of atrial cells 211b and the center cells 211e helping transition from rows of twelve cells on the atrial side to rows of twenty-four cells on the ventricular side. A second row of center cells 211f may be positioned at a longitudinal center of the frame 200, each center cell 211f being positioned between an atrial cell 211b and center cell 211e. In the illustrated embodiment, center cells 211f in the second row may be diamond-shaped, with the second row including twenty-four center cells 211f. Finally, a third row of center cells 211g may be positioned between the second row of center cells 211f and the second row of ventricular cells 211d. The third row of center cells 211g may include twenty-four cells and they may each be substantially diamond-shaped. However, it should be understood that the particular number, shape, and configuration of center cells may be different than the specific embodiment shown.

All of the cells 211a-g may be configured to expand circumferentially and foreshorten axially upon expansion of the frame 200. A pin or tab 222 may extend from an apex of each atrial cell 211a in the first row in a direction toward the outflow end of the frame 200. Although one pin or tab 222 is illustrated in each atrial cell 211a in the first row, in other embodiments fewer than all of the atrial cells in the first row may include a pin or tab. These pins or tabs 222 may be configured to receive a suture or suture loop of a delivery device so that the frame 200 (and thus the prosthetic heart valve 100) remains coupled to the delivery system until the user decouples the suture loops from the pins or tabs 222.

In some embodiments, frame 200 may include a plurality of tines or barbs 208 extending from a center portion or ventricular portion of the frame for piercing or otherwise engaging native tissue in the native annulus or in the native leaflets. In the illustrated embodiment, each barb 208 is connected to a ventricular cell 211d in the second row. In some embodiments, the barb 208 may be coupled to an inflow or outflow apex of each cell. In the particular illustrated embodiment, the barbs 208 are coupled to ventricular cells 211d on an inflow half of the cell, on either side of the inflow apex. For example, the barb 208 in one ventricular cell 211d may be coupled to the inflow half of that cell on a right side of the apex, with the adjacent ventricular cell 211d having a barb coupled to the inflow half of that cell on a left side of the apex. With this configuration, the barbs 208 are provided in pairs with relatively little space between the barbs of a pair, but a relatively large space between adjacent pairs. However, it should be understood that the barbs 208 may in other embodiments be centered with even spacing between adjacent barbs. In the collapsed condition of the frame 200, each barb 208 extends toward the outflow end of the frame, each barb being positioned within a ventricular cell 211d in the second row. In the expanded condition of the frame 200, the barbs 208 may hook upwardly back toward the inflow end, the barbs being configured to pierce native tissue of the valve annulus, such as the native leaflets, to help keep the prosthetic heart valve from migrating under pressure during beating of the heart. However, in some embodiments, the tines or barbs 208 may be completely omitted. For example, the tines or barbs 208 may be particularly helpful when used in a native mitral valve, as a prosthetic mitral valve must withstand relatively high pressures, and the tines or barbs 208 may assist with anchoring. However, the tines or barbs 208 may be omitted when the prosthetic heart valve is used as a prosthetic tricuspid valve, as pressures within the right heart are significantly lower than pressures within the left heart, and thus the tines or barbs 208 may not be needed at all for anchoring. In fact, the tines or barbs 208 may increase the likelihood of conduction disturbances, and particularly in the context of a prosthetic tricuspid valve, it may be preferable to omit the tines or barbs 208 entirely.

In addition to the frame 200, a typical prosthetic atrioventricular valve may include an inner metal frame to which prosthetic leaflets are attached. However, referring back to Fig. 2, prosthetic heart valve 100 may completely omit any inner metallic or otherwise rigid frame to which the prosthetic leaflets are attached. For example, in the particular embodiment shown in Fig. 2, prosthetic heart valve 100 omits an inner metallic or otherwise rigid frame, and instead includes a soft tube 300 that is secured to the frame 200. The tube 300 is formed of any suitable biocompatible material, excluding metallic materials, but including for example PET, PTFE, ePTFE, UHMWPE, *etc.,* including in a fabric form or another form, including extruded or flat sheet polymers. In some examples not forming part of the invention, the tube 300 may be formed of tissue, such as bovine or porcine pericardium. Preferably, the material (*e.g.*, fabric or tissue) that forms tube 300 is substantially fluid-tight or otherwise substantially impermeable to blood so that blood is only able to flow through the lumen of the tube 300, and not through the wall(s) that form the tube 300. A plurality of prosthetic leaflets 400 may be coupled directly to the tube 300, for example by suturing. In the illustrated embodiment of Fig. 2, the prosthetic heart valve 100 includes three leaflets 400, with each leaflet including a free edge 410 and an attached edge 420. In operation, the free edges 410 move away from each other to allow for blood to flow through the tube 300 and the leaflets 400 in the antegrade direction *(i.e.,* from the atrium to the ventricle), and coapt together to restrict blood from flowing through the tube 300 in the retrograde direction *(i.e.,* from the ventricle to the atrium). The attached edge 420 may be opposite the free edge 410 and may be attached to the tube 300 via any suitable mechanism, including fasteners such as sutures. In the illustrated embodiment, the attached edges 420 generally follow a "U"-shaped, catenary, or generally parabolic pattern. Preferably, the prosthetic leaflets 400 are formed of bioprosthetic tissue, such as bovine or porcine pericardium, but in other embodiments, the prosthetic leaflets 400 may be formed of fabrics or other synthetic materials, such as PET, PTFE, UHMWPE, *etc.*

Still referring to Fig. 2, the prosthetic heart valve 100 may include a covering or lining, such as a skirt 500. Skirt 500 may be formed of any suitable material, including tissue, fabric, or extruded or flat sheet polymers. For example, skirt 500 may be formed of a woven synthetic fabric such as PET, PTFE, UHMWPE, *etc.* that functions to contact native tissue at or adjacent to the native heart valve annulus and provide a conforming seal. For example, skirt 500 may include an outer or peripheral section 510 attached to the luminal or abluminal (as shown in Fig. 2) surface of the frame 200, for example via suturing. Upon implantation of the prosthetic heart valve 100, the outer section 510 of the skirt 500 preferably contacts the native valve annulus to help seal against the native valve annulus and to prevent paravalvular leakage around the prosthetic heart valve 100. The skirt 500 may also include an outflow section 520 generally extending radially inwardly from the frame 200 to the tube 300. The outflow section 520 may be formed of the same or different materials as the peripheral or outer section 510 of the skirt 500, and for example, may be substantially impermeable to blood flowing therethrough. The outflow section 520 in the embodiment shown in Fig. 2 is substantially planar and has an annular shape, with the outer circumference or perimeter of the outflow section 520 coupled to the frame 200 (*e.g.,* via sutures) and the inner circumference or perimeter of the outflow section 520 coupled to the outflow end of the tube 300 (*e.g.,* via sutures). The skirt 500 may also include an inflow section that is substantially similar to the outflow section, with the exception that it is positioned on the inflow section of the prosthetic heart valve 100 (which is not visible in the view of Fig. 2). Thus, the inflow section may be generally annular with an outer perimeter or circumference attached to the frame 200 and an inner perimeter of circumference attached to the outer perimeter of the inflow end of tube 300.

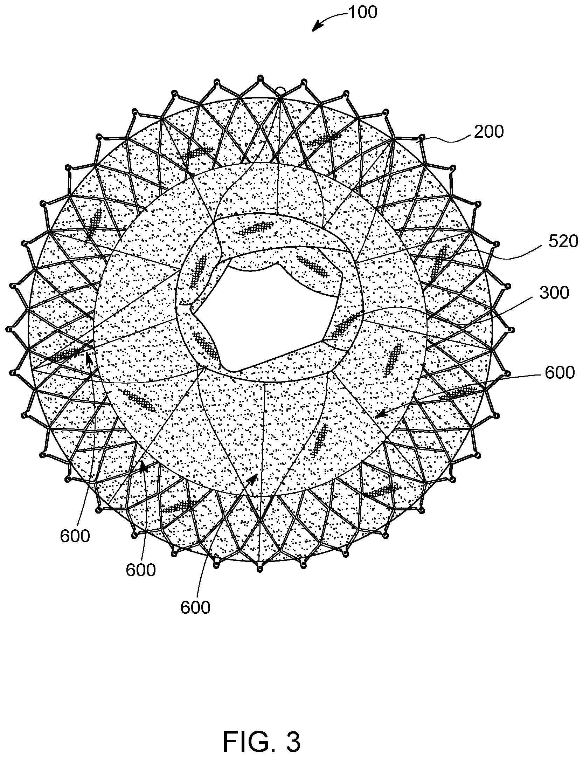

Fig. 3 shows the prosthetic heart valve 100 of Fig. 2 prior to the prosthetic leaflets 400 being attached to the tube 300. Fig. 3 shows particularly well one mechanism by which the tube 300 may be coupled to the prosthetic heart valve 100. For example, although the inflow and outflow ends of the tube 300 may be coupled to the inner perimeters of the inflow and outflow sections of skirt 500, those couplings may be primarily to help ensure that blood only flows through the lumen of tube 300. It may be desirable to provide additional structural support for the tube 300, particularly since the prosthetic leaflets 400 therein will need to resist significant forces when they close during ventricular systole. As shown in Fig. 3, one or more connectors 600 may directly couple the tube 300 to the frame 200. For example, the embodiment of Fig. 3 illustrates a plurality of radially extending sutures 600, each suture 600 having a first end coupled to the frame 200 and a second end coupled to the tube 300, with the suture 600 generally extending along a line that would pass through (or nearly pass through) a radial center of the tube 300. In the illustrated embodiment, one group of sutures 600 is provided at the outflow end of the prosthetic heart valve 100, and although not shown, a generally similar or identical group of suture connectors 600 is provided at the inflow end of the prosthetic heart valve 100, so that both the inflow and outflow ends of the tube 300 are secured to the frame 200 via radially extending sutures 600. The suture connectors 600 may only provide support in tension, and thus help to minimize the movement of the inner valve *(e.g.,* the tube 300 with the prosthetic leaflets 400 coupled thereto) during normal operation of the prosthetic heart valve 100. On the other hand, when the frame 200 is compressed (e.g., when being collapsed for delivery, or when the native tissue applies force to the frame 200 during the normal cycle of the heart), that compressive force is not translated via the suture connectors 600 to the tube 300. In some embodiments, a tensioning mechanism may be provided with one or more of the suture connectors 600 to allow for adjusting the tension of the suture connectors 600 during or after delivery and deployment of the prosthetic heart valve 100. One example of a suitable tensioning mechanism is a ratcheting mechanism, with a portion of the suture connectors having a feature that slides against the connection point in only one direction to tighten. Alternatively, a mechanism that knots, swages, or pins at the connection point may be used when the desired tension and/or annular motion is reached. It should be understood that, although the term "suture connector" is used herein, the connectors are not actually limited to sutures - but may be any suitable cord, string, or wire-like material that is sufficiently strong to provide the desired support to the inner valve. Further, it should be understood that suture connectors 600 may be only one example of how the tube 300 may be coupled to the frame 200. For example, instead of using suture connectors 600, rigid arms, such as arms formed of nickel-titanium alloy or Nitinol, may be incorporated into the frame 200 with the arms being bent inwardly to form a general "C" shape with the ends of the "C" shape coupled to the top and bottom, respectively, of the tube 300. Examples of suitable arm connectors are described in greater detail in U.S. Patent Application No. 18/067,993 titled "Two Stage Tricuspid Valve Implant" and filed on December 19, 2022. Such connector arms may provide more rigid support than the suture connectors 600.

As noted above, prosthetic heart valve 100 of the present invention lacks a metallic inner frame for supporting the prosthetic leaflets 400 that is frequently found in collapsible and expandable prosthetic atrioventricular valves. By eliminating this metallic inner frame, the prosthetic heart valve 100 of the present invention is able to collapse to a smaller size (*e.g.,* a smaller French size) and thus a smaller catheter may be used to deliver the prosthetic heart valve 100, compared to an otherwise similar prosthetic heart valve that includes a metallic or otherwise rigid inner frame. It is generally desirable to use smaller catheters, when possible, to deliver a prosthetic heart valve via a transvascular route since larger catheters may present a greater risk to the patient, particularly at the access site (*e.g.,* the femoral vein). This design may also reduce the forces required to load the prosthetic heart valve into the delivery device. In other words, when collapsing the prosthetic heart valve 100 to the collapsed condition for storage within a delivery device for the procedure, a smaller force may be required to collapse the valve which is generally desirable. Still, other benefits may arise from the single-frame design of prosthetic heart valve 100. For example, retrieving a prosthetic heart valve after it has been partially or completely deployed into the native valve annulus can be very difficult when two separate rigid frames are used. The use of two separate rigid frames may increase the forces required to retrieve (*e.g.*, by re-collapsing) the prosthetic heart valve, and the existence of two spaced apart rigid frame structures may create a greater likelihood of frame structure getting "caught" on a retrieval catheter as the prosthetic heart valve is being re-collapsed into the retrieval catheter. Forming the prosthetic heart valve 100 with only a single rigid frame may reduce or eliminate both of these potential issues. Still another potential benefit of the single frame design of prosthetic heart valve 100 is that, because there is no rigid connection between the prosthetic leaflets 400 and the frame 200 that is directly in contact with the native valve, any deformation of the frame 200 during normal operation of the prosthetic heart valve 100 is highly unlikely to result in any deformation of the prosthetic leaflets 400. Deformation of the prosthetic leaflets 400 is undesirable because any deformation to the shape of the prosthetic leaflets 400 during operation may negatively affect the ability of the prosthetic leaflets 400 to properly coapt and to create a complete seal during ventricular systole. In other words, if the deformation of the frame 200 caused deformation of the prosthetic leaflets 400, the prosthetic heart valve 100 may allow for undesirable regurgitation across the prosthetic leaflets 400.

Although prosthetic heart valve 100 is described above as having prosthetic leaflets 400 directly attached (*e.g*., via sutures) to the tube 300, in some embodiments, additional support materials may be provided at the leaflet-tube interface. For example, an underwire type of structure may be attached to the tube 300, and portions of the prosthetic leaflets 400 attached to the underwire, to provide additional support. The underwire may take the form of wire, such as a strand of Nitinol, that has a general "U"-shape corresponding to each prosthetic leaflet 400. For example, referring back to Fig. 2, a Nitinol underwire that follows the general contours of the attachment edge 420 may be interposed between the prosthetic leaflets 400 and the tube 300. In some embodiments, the underwire may be substantially continuous so that the prosthetic leaflets 400 are attached to the underwire along the leaflet bellies (*e.g.,* along attachment edge 420), as well as the commissures where adjacent prosthetic leaflets 400 join. In some embodiments, only the leaflet bellies may be attached to an underwire provided on the tube 300. In some embodiments, only the leaflet commissures may be attached to an underwire (or another support structure, such as a commissure plate) on the tube 300. It should be understood that these additional support structures may offer a compromise in the sense that, while the additional metal (or otherwise rigid) material on the tube 300 may increase bulk, it may only increase bulk slightly compared to the use of a full inner metal stent, but the additional support to the prosthetic leaflets 400 may justify the additional bulk created.

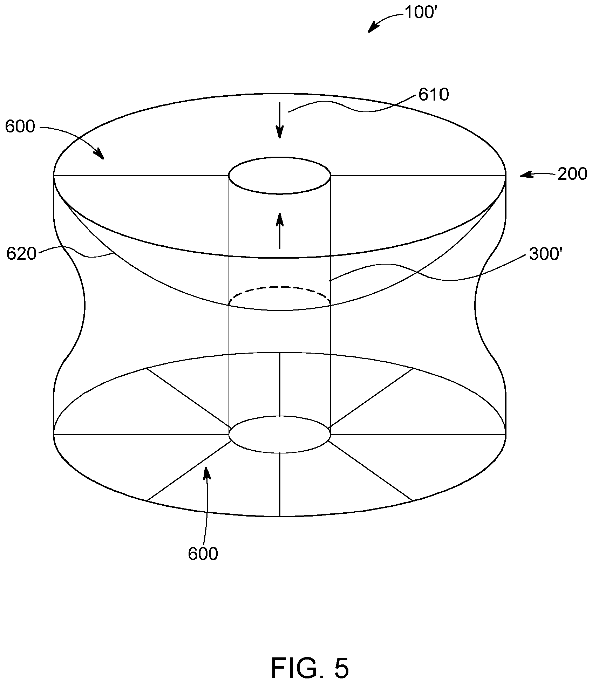

Although prosthetic heart valve 100 is described and shown in connection with Figs. 2-4 as including prosthetic leaflets 400 attached to the tube 300, in some embodiments, the tube itself may function as a valve without the need for separate leaflets. For example, Fig. 5 illustrates a prosthetic heart valve 100' that is similar to prosthetic heart valve 100 in a number of respects. For example, prosthetic heart valve 100' may include frame 200, which may be similar or identical to frame 200 described above. However, as with prosthetic heart valve 100, the frame 200 of prosthetic heart valve 100' may take other suitable forms besides the particular features described in connection with frame 200. In addition, prosthetic heart valve 100' may include a tube 300' of soft material, such as tissue or fabrics as described in connection with tube 300'. The main difference between prosthetic heart valve 100 and 100' is that the tube 300' itself provides the valving functionality, without the need for separate leaflets. In particular, the inflow end of tube 300' (toward the bottom in the view of Fig. 5) may be coupled to the frame 200 in substantially the same fashion as described in connection with prosthetic heart valve 100. In particular, a plurality of connectors 600, which may be suture or suture-like connectors 600, extend radially outwardly from the inflow end of the tube 300' to the frame 200 to provide connection points therebetween. Each suture connector 600 may have a first end coupled to the inflow end of the tube 300' and a second end coupled to the frame 200, with the suture connectors 600 generally extending in a direction toward the radial center of the tube 300'. Preferably, enough suture connectors 600 are provided on the inflow end of the tube 300' to ensure that the inflow end of the tube 300' cannot close or otherwise collapse on itself. For example, 4, 5, 6, 7, 8, 9, or more suture connectors 600 may connect the inflow end of the tube 300' to the frame 200. Preferably, the suture connectors 600 are positioned at substantially equal intervals around the circumference of the inflow end of the tube 300'. The outflow end of the tube 300', however, has fewer suture connectors 600. In the illustrated embodiment, the outflow end of the tube 300' has exactly two suture connectors 600 that connect the outflow end of the tube 300' to the frame 200, with the two suture connectors 600 being positioned at diametrically opposed points of the outflow end of the tube 300'. With this configuration, the two suture connectors 600 at the outflow end of the tube 300' will maintain the position of the connection points of the tube 300' relative to the frame 200. However, because the outflow end of the tube 300' excludes additional suture connectors 600, the unconnected or free edges of the outflow end of the tube 300' are generally free to open and close depending on the pressure gradient across the prosthetic heart valve 100'. For example, during atrial systole, the higher pressure in the atrium will force blood to flow through the tube 300', with the inflow end of the tube 300' being restricted from closing due to the various suture connectors 600, and the outflow end of the tube 300' naturally "wanting" to remain open because of the pressure gradient. However, during ventricular systole, the higher pressure in the ventricle will tend to cause the outflow end of the tube 300' to collapse where the suture connectors 600 allow for such collapsing. In the illustrated embodiment of Fig. 5, arrows 610 illustrate that the outflow end of the tube 300' will tend to close on each other on either side of the pair of suture connectors 600, much like a duckbill valve. In other words, if the suture connectors 600' on the outflow end of the tube 300' are thought of as being positioned at 3 o'clock and 9 o'clock, the outflow end of the tube 300' will tend to close along the 6 o'clock and 12 o'clock directions when the pressure in the ventricle is greater than the pressure in the atrium.

Still referring to Fig. 5, the tube 300' may be formed as a single generally cylindrical piece of fabric or tissue (*e.g.,* tissue that is rolled into a tube shape), or two separate pieces of fabric or tissue sewn together with opposing seams. If the tube 300' is formed as two pieces of material sutured together at opposing seams (*e.g.,* seams running vertically or longitudinally), it may be preferable for the suture connectors 600 at the outflow end of the tube 300' to connect at or near the seams, so that the outflow end of the tube 300' opens and closes between the seams.

Although not shown in detail in Fig. 5, prosthetic heart valve 100' may include a skirt substantially similar or identical to skirt 500. For example, as shown in Fig. 5, a skirt 620 may be generally at the outflow end of prosthetic heart valve 100', but with a center portion of the skirt 620 angled or oriented toward the inflow end, with the tube 300' extending through a center portion of the skirt 620. In other embodiments, the skirt 620 at the outflow end could be omitted. In either case a skirt may be provided at the inflow end of the prosthetic heart valve 100' as well. In other embodiments described herein, the outflow skirt (*e.g.* skirt 500) is provided at the outflow most end of the leaflet assembly, or even downstream of the leaflets. However, because the outflow end of the tube 300' acts as the valve, and includes edges that are in motion, the outflow skirt 620 cannot be directly attached to the outflow end of the tube 300', as such connection could interfere with the opening and closing of the outflow end of the tube 300'. Thus, the center portion of the skirt 620 is coupled to the tube 300' at a spaced distance away from the outflow end of the tube 300' to allow the outflow end of the tube 300' to open and close during normal operation.

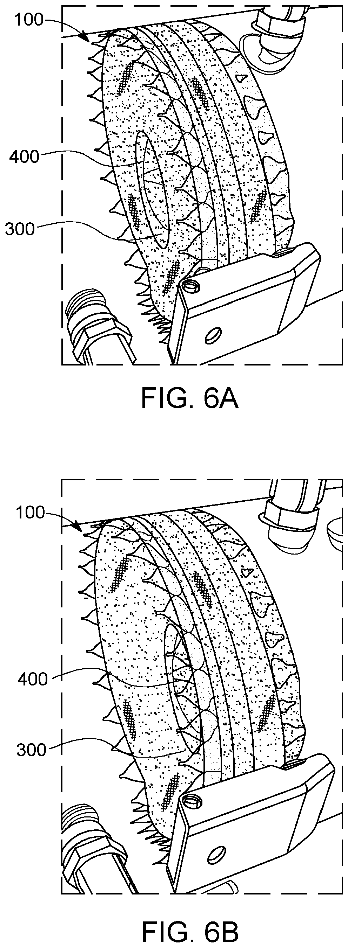

One possible result of excluding a rigid inner frame, or otherwise any rigid attachment between the prosthetic leaflets 400 and the frame 200, is that pulsatile motion of the prosthetic leaflets 400 may occur during normal operation of prosthetic heart valve 100. For example, Figs. 6A-B illustrate the prosthetic heart valve 100 deployed into a test system that simulates flow through the prosthetic heart valve 100. In the view of Figs. 6A-B, the inflow side of the prosthetic heart valve 100 is toward the right of the views and the outflow side of the prosthetic heart valve is toward the left of the views. Fig. 6A illustrates a portion of the test in which the fluid pressure on the inflow (right) side of the prosthetic heart valve 100 is greater than on the outflow (left) side of the prosthetic heart valve 100, forcing the prosthetic leaflets 400 to open to allow fluid to flow through the tube 300. Fig. 6B illustrates a portion of the test in which the fluid pressure on the outflow (left) side of the prosthetic heart valve 100 is greater than on the inflow (right) side of the prosthetic heart valve 100, forcing the prosthetic leaflets 400 to close to prevent fluid from flowing backward through the tube 300. As can be seen by comparing Figs. 6A-B, when the leaflets 400 are open and fluid is flowing from the inflow (right) to the outflow (left) end of the prosthetic heart valve 100, the prosthetic leaflets 400 and tube 300 are positioned relatively far in the outflow direction. On the other hand, when the leaflets 400 are closed and are preventing fluid from flowing from the outflow (left) to the inflow (right) end, the prosthetic leaflets 400 and tube 300 are positioned relatively far in the inflow direction. Despite the movement of the prosthetic leaflets 400 and the tube 300, the frame 200 remains in the same (or substantially the same) position during the simulated cycle of the heart. The extent of motion may be represented as a fraction of the inflow-to-outflow length of the tube 300. For example, the tube 300 may have a length in the axial direction between the inflow and outflow ends, and the maximum displacement of the tube 300 during a single cycle of the heart may be between about 20% and about 60%, including about 30%, about 40%, or about 50% of the length of the tube 300. In a healthy heart, there is typically relative motion between the ventricular wall and the valve annulus. The above-described features may allow for this natural relative motion to be recreated, whereas in other implant features, the relative motion might not be recreated.

Prosthetic heart valves intended for use in replacing a tricuspid *(i.e.,* right atrioventricular) valve may include additional or alternative features than those described above particularly suited for use in the tricuspid space. For example, one concern that is of particular interest with tricuspid valve replacements is the fact that the AV node is typically located within the right atrium, and prosthetic tricuspid valves with an atrial cuff may be at risk of pressing against the AV node which may disturb the natural conduction system of the heart. Further, the tricuspid valve annulus is typically (but not necessarily always) larger than the annuli of the remaining heart valves (mitral, aortic, and pulmonary). Thus, while anchoring is almost always a relevant concern for a prosthetic heart valve, the concern may be heightened in the case of a prosthetic tricuspid valve. Various prosthetic tricuspid valves are described below which may address one or both of the above-noted issues.

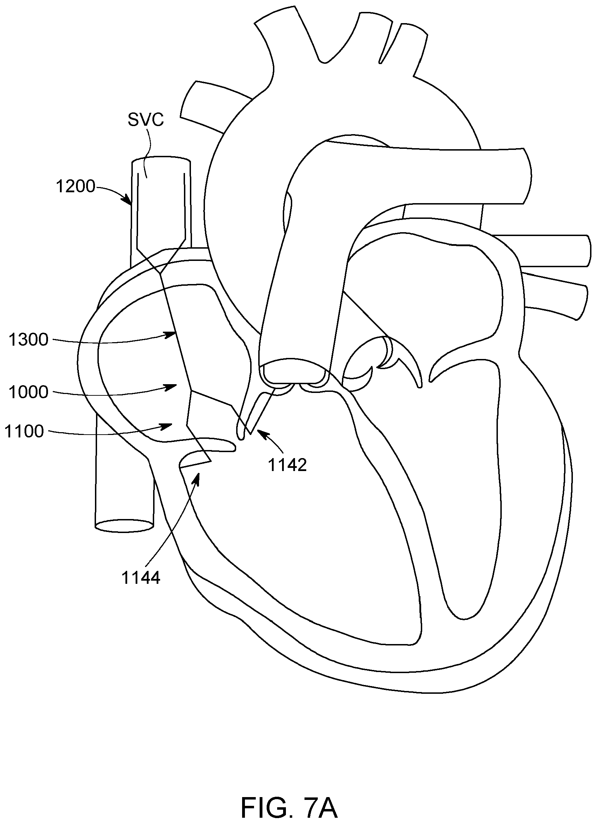

Fig. 7A illustrates a highly schematic view of a prosthetic tricuspid valve system 1000 implanted in a native tricuspid valve, with the heart being shown in a cutaway view. The prosthetic tricuspid valve system 1000 may generally include three components, including a prosthetic valve 1100 to provide the replacement valve functionality, an anchor 1200 to serve as an anchor point for the prosthetic tricuspid valve system 1000, and a connecting line or tether 1300 to couple the prosthetic valve 1100 to the anchor 1200.

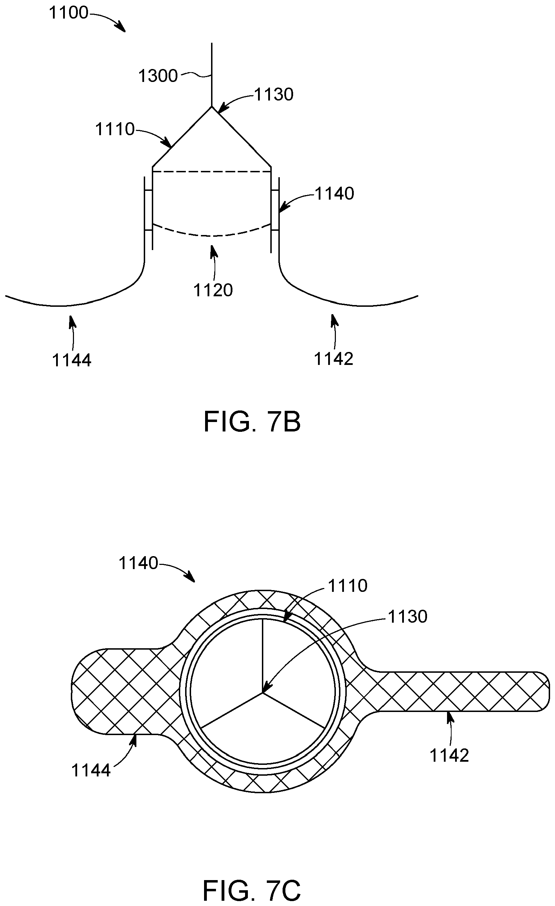

Fig. 7B is an isolated schematic view of the prosthetic valve 1100 of the valve system 1000. The prosthetic valve 1100 may include a collapsible and expandable stent or frame 1110. Frame 1110 may be formed of a shape memory metal or metal alloy such as Nitinol, and may be formed as a braided mesh or an integral member, for example laser cut from a tube of Nitinol and then heat treated to establish the desired shape in the expanded condition. The frame 1110, when in the expanded condition shown in Fig. 7B, may include a main portion that is generally cylindrical, a set of prosthetic leaflets 1120 being received within and coupled to the main portion. The prosthetic leaflets 1120 may be substantially similar or identical to prosthetic leaflets 400 described above. It should be understood that, in the view of Fig. 7B, the outflow end is toward the bottom of the view while the inflow end is toward the top of the view. At the inflow end of the prosthetic heart valve 1100, the main portion of the frame 1110 may transition to a connector 1130, the connector 1130 coupling the tether 1300 to the frame 1110. Briefly referring to Fig. 7C, the transition may include a plurality (e.g., three) of individual struts that have first ends connected to the main portion of the frame 1110 and which converge to a central portion which is the connector 1130. In some embodiments, the connector 1130 is not a separate structure and is simply the point of fixation between the tether 1300 and the frame 1110. However, specialized features may be provided for connector 1130, an example of which is described in greater detail below.

Referring back to Fig. 7B, the prosthetic valve 1100 may also include an outer frame 1140 coupled to the inner frame 1110. In some embodiments, the outer frame 1140 may be sutured or otherwise fastened to the inner frame 1110. In other embodiments, the outer frame 1140 and the inner frame 1110 may not be separate structures, but rather formed integrally, for example via laser cutting from a single tube of Nitinol. The prosthetic valve 1100 may be asymmetric and have a single intended orientation for implantation. The outer frame 1140 may include two main valve anchoring features, including a hook 1142 and a shelf 1144. The hook 1142 may be referred to as a right ventricular outflow tract ("RVOT") hook 1142, and is intended to hook over the outflow side of the native tricuspid valve leaflets adjacent to the RVOT, which is generally the area through which blood flows from the right ventricle through the pulmonary valve. The intended position of the RVOT hook 1142 is best shown in Fig. 7A. In the expanded or deployed condition, the RVOT hook 1142 may extend from the main portion of the frame 1110 in the outflow direction, extending radially outward from the center of the prosthetic valve 1100 and then hooking back toward the inflow direction of the prosthetic valve 1100. The shelf 1144, which may be referred to as a posterior shelf, is generally similar in overall shape and structure to the RVOT hook 1142, but extends from the diametrically opposed portion of the prosthetic valve 1100, as best shown in Figs. 7A and 7C. As with RVOT hook 1142, posterior shelf 1144 is positioned on the outflow or ventricular side of the native tricuspid valve after deployment, but extends generally toward the right ventricular wall (the ventricular wall opposite the intraventricular septum). Also, as with RVOT hook 1142, posterior shelf 1144 may extend from the main portion of the frame 1110 in the outflow direction, extending radially outward from the center of the prosthetic valve 1100 and then hooking back toward the inflow direction of the prosthetic valve 1100.

Referring again to Fig. 7C, the structure and shape of the outer frame 1140 are shown and described in greater detail below. Fig. 7C is a top view of prosthetic heart valve 1110, as viewed from the inflow-to-outflow direction. In the embodiment of Fig. 7C, the outer frame 1140 is a separate component from the frame 1110, and generally surrounds the frame 1110 and is fastened to the frame 1110 *(e.g.,* via sutures). The portions of the outer frame 1140 between the RVOT hook 1142 and the posterior shelf 1144 *(e.g.,* the top and bottom portions in the view of Fig. 7C) may be relatively thin and mainly intended to provide a structure that connects to the RVOT hook 1142 and posterior shelf 1144. The RVOT hook 1142 may extend a distance radially away from the center of the prosthetic heart valve 1100 greater than the radial distance that the posterior shelf 1144 extends from the center of the prosthetic heart valve 1100. However, the RVOT hook 1142 may be narrower compared to the posterior shelf 1144. In particular, the width of the posterior shelf 1144, measured in a direction orthogonal to the flow direction of the prosthetic valve 1100 and perpendicular to the directions in which the RVOT hook 1142 and posterior shelf 1144 extend, may be greater than the width of the RVOT hook 1142 measured in the same direction. The RVOT hook 1142 and posterior shelf 1144, when the prosthetic heart valve 1100 is deployed in the native tricuspid valve annulus, mainly function to provide a force that counters the tension from tether 1300, described in greater detail below. In other words, although the RVOT hook 1142 and posterior shelf 1144 may assist with sealing the prosthetic valve 1100 against the native tricuspid valve annulus, the main purpose is to provide an anchoring force against migration of the prosthetic heart valve 1100 into the right atrium. Although not shown, the prosthetic heart valve 1100 may include any suitable skirt or sealing member on outer surfaces thereof to contact the native anatomy to help with sealing against paravalvular leak.

As should be understood from Figs. 7A-C and the corresponding description, the only anchoring features of the prosthetic heart valve 1100 that result in direct contact with the native tricuspid valve annulus are the RVOT hook 1142 and posterior shelf 1144, which contact the outflow or ventricular side of the native tricuspid valve. Also, to the extent that the self-expansion force of the main body of the prosthetic heart valve 1100 in the native tricuspid annulus provides additional anchoring, the contact is generally only with the inner surface of the native tricuspid valve annulus. One benefit is that the prosthetic heart valve 1100 may be anchored within the native tricuspid valve without any atrial cuff that is typical of a prosthetic tricuspid valve. In other words, there is no flared atrial end that sits in contact with the inflow or atrial side of the prosthetic tricuspid valve. As noted above, the AV node is typically located on the atrial side of the native tricuspid valve annulus near the atrial septum. The anchoring described and shown in connection with Figs. 7A-C completely avoids contact with the AV node, reducing the likelihood of conduction disturbances that might result in the prosthetic heart valve 1100 contacting or otherwise pressing against the AV node.

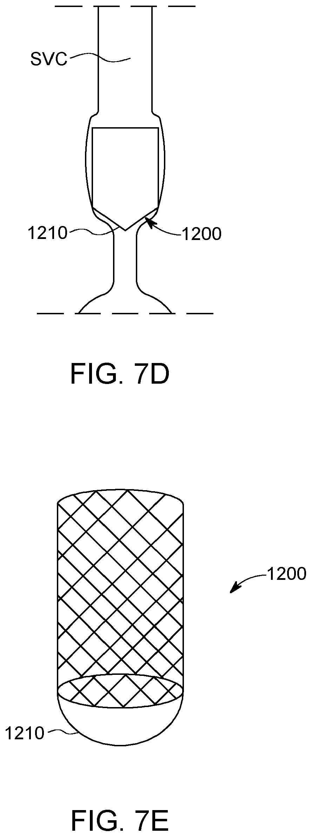

Fig. 7D illustrates the anchor 1200 of the tricuspid valve system 1000 deployed within the superior vena cava SVC. Anchor 1200 may take the form of a collapsible and expandable stent. In some embodiments, the anchor 1200 may be balloon expandable and formed of a plastically expandable material such as stainless steel or cobalt chrome. In other embodiments, the anchor 1200 may be self-expandable and formed of a shape memory material such as Nitinol. Preferably, anchor 1200 is generally cylindrical when expanded and thus does not disrupt (or does not materially disrupt) the flow of blood through the superior vena cava SVC. If the anchor 1200 is self-expandable, the anchor 1200 is preferably oversized so that, in the absence of applied forces, the outer diameter of the anchor 1200 is larger than the inner diameter of the superior vena cava SVC. In other words, the anchor 1200 may be "oversized" relative to the superior vena cava SVC so that, when the anchor 1200 self-expands into the superior vena cava SVC, the anchor 1200 at least slightly deforms the shape of the superior vena cava SVC to help prevent axial migration of the anchor 1200. This local deformation of the superior vena cava SVC can be seen in Fig. 7D. If the anchor 1200 is balloon expandable, the balloon (or other mechanism that forces the anchor 1200 to radially expand), may be used to expand the anchor 1200 until it has a diameter that is slightly larger than the natural inner diameter of the superior vena cava SVC.

Still referring to Fig. 7D, although the anchor 1200 may be generally cylindrical in the expanded condition, the anchor preferably includes a feature to assist with the connection of the tether 1300 to the anchor 1200. For example, as shown in Fig. 7D, the outflow end of the anchor 1200 (which is positioned closest to the right atrium upon deployment into the superior vena cava SVC) may include an arch 1210 that the tether 1300 may be looped around, as described in greater detail below. Fig. 7E illustrates the anchor 1200 in isolation in an expanded condition, showing the arch 1210 that extends beyond the outflow end of the main cylindrical portion of the anchor 1200. In the particular example, arch 1210 may be formed as a single strut or strand of metal that has ends coupled to diametrically opposed points of the anchor 1200, similar to a handle of a bucket. Although arch 1210 is shown as a generally arcuate member, in some embodiments it may be more "V"-shaped which may assist with the arch 1210 more easily collapsing for delivery. It should be understood that other shapes and configurations of arch 1210 may be suitable. And although arch 1210 is described in connection with looping of the tether 1300 around the arch 1210 to connect the tether 1300 to the anchor 1200, as described in greater detail below, it should be understood that any mechanism for connecting the tether 1300 to the anchor 1200 may be suitable.

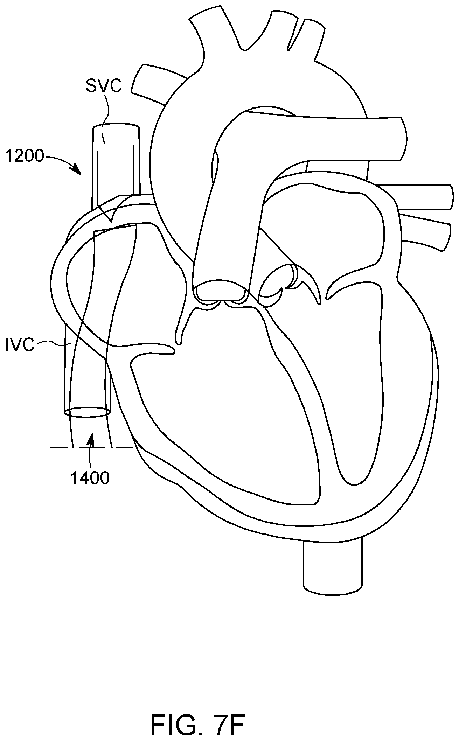

Figs. 7F-H illustrate different stages in the delivery and deployment of prosthetic tricuspid valve system 1000 into a patient's heart. During an exemplary delivery and deployment of the prosthetic tricuspid valve system 1000, the anchor 1200 may be loaded into a catheter 1400 of a delivery device in a collapsed condition. The catheter 1400 may be passed into the patient, for example through an access site in the femoral vein, and the catheter 1400 may be advanced through the inferior vena cava IVC, into the right atrium, and then into the superior vena cava SVC. When the distal end of the catheter 1400 has reached the desired distance within the superior vena cava SVC, the anchor 1200 may be deployed from the catheter 1400 and transitioned into the expanded condition shown in Fig. 7F. In one example, the anchor 1200 may be loaded over an inflatable balloon and the balloon may be inflated to force the anchor 1200 to expand into the superior vena cava SVC. In another example, the anchor 1200 may be advanced distally relative to the distal end of the catheter 1400, and the anchor 1200 will self-expand as the catheter 1400 uncovers the anchor 1200. The mechanism by which the anchor 1200 is advanced relative to the catheter 1400 may be any suitable mechanism. For example, an interior pusher may be pushed distally to push the anchor 1200 out of the catheter 1400. In another embodiment, the anchor 1200 may be releasably coupled to an inner catheter shaft, and the catheter 1400 may be withdrawn proximally relative to the anchor 1200 until the anchor 1200 self-expands away from the inner catheter shaft. With either option, the anchor 1200 is preferably expanded to a size that has a larger diameter than the natural inner diameter of the superior vena cava SVC, as described above, with the arch 1210 facing in the outflow direction *(i.e.,* toward the right atrium). In this embodiment, the stent 1200 is delivered in isolation without being connected to either the tether 1300 or the prosthetic heart valve 1100 at the time of deployment of the stent 1200.

After the anchor 1200 is deployed, the prosthetic valve 1100 may be delivered and deployed next. In some embodiments, the same catheter 1400 that delivered the anchor 1200 may be used to deliver the prosthetic valve 1100. For example, the prosthetic valve 1100 may be pre-loaded into the catheter 1400 in a collapsed condition in a position proximal to the anchor 1200. In other embodiments, the catheter used to deliver the prosthetic valve 1100 may be a separate catheter. Although either option is feasible, for brevity, the same part number 1400 is used to describe the catheter that delivers the prosthetic heart valve 1100. In either embodiment, after the anchor 1200 is deployed satisfactorily, the catheter 1400 may be positioned or re-positioned so that the distal end of the catheter 1400 is at or adjacent to the native tricuspid valve. When loaded into the catheter 1400, the prosthetic valve 1100 is oriented so that the RVOT hook 1142 and the posterior shelf 1144 are at the leading end of the prosthetic valve 1100. Preferably, while loaded into the catheter 1400, the RVOT hook 1142 and posterior shelf 1144 do not radially overlap the main body of frame 1110.

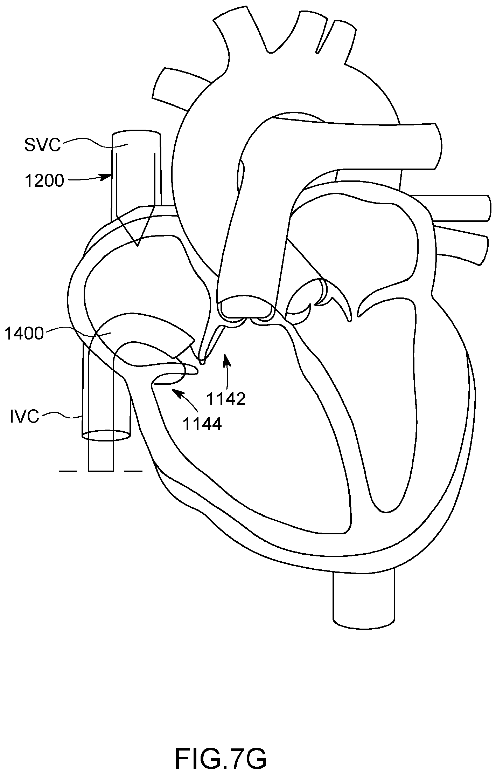

As best shown in Fig. 7G, once the catheter 1400 is at the desired position relative to the native tricuspid valve, the prosthetic heart valve 1100 may be deployed. Similar to the anchor 1200, the deployment of the prosthetic heart valve 1100 may include withdrawing the catheter 1400 while the prosthetic heart valve 1100 maintains its position, pushing the prosthetic heart valve 1100 distally out of the catheter 1400, or a combination of the two. The first portions of the prosthetic heart valve 1100 that exit the catheter 1400 are the RVOT hook 1142 and the posterior shelf 1144. As they exit the catheter 1400, and the catheter 1400 no longer constrains them, the RVOT hook 1142 and posterior shelf 1144 will tend to revert to their shape-set conditions, causing the RVOT hook 1142 and posterior shelf 1144 to "hook" backward after exiting the catheter 1400. As the RVOT hook 1142 and posterior shelf 1144 hook backward during deployment, they hook over the outflow portion of the native tricuspid valve annulus, which may include native tricuspid valve leaflets.

Prior to describing the remaining portions of the exemplary delivery and deployment procedure, the tether 1300 is described briefly. Tether 1300 may be in the form of any string-like or wire-like structure that is biocompatible and is capable of withstanding tension that would otherwise tend to push the prosthetic heart valve 1100 into the right ventricle. For example, tether 1300 may be a metal structure, such as a monofilament or a multifilament, including for example Nitinol. Tether 1300 may alternatively be formed of a polymer, such as one or more strands or filaments or threads of PE, PTFE, UHMWPE, *etc.* In one exemplary embodiment, the tether 1300 is formed as a braided polymer. The tether 1300 may include a first end portion that is fixed to the prosthetic valve 1100 prior to the prosthetic heart valve 1100 being loaded into the catheter 1400. For example, the connector 1130 of the frame 1110 may include a generally cylindrical stent section which may be sized to receive an end of the tether 1300, with the connector 1130 being clamped over and/or fastened (e.g., by sutures) to the tether 1300 positioned therein. Examples of connectors 1130 for receiving tethers are described in greater detail in U.S. Patent No. 10,405,976. Thus, while the prosthetic heart valve 1100 is within the catheter 1400 being delivered, the tether 1300 may already be coupled or otherwise fixed to the prosthetic heart valve 1100 with the tether 1300 trailing (or being positioned generally proximal to) the prosthetic heart valve 1100. The tether 1300 may have a length to extend to a handle of a delivery device or beyond a handle during the delivery of the prosthetic heart valve 1100.

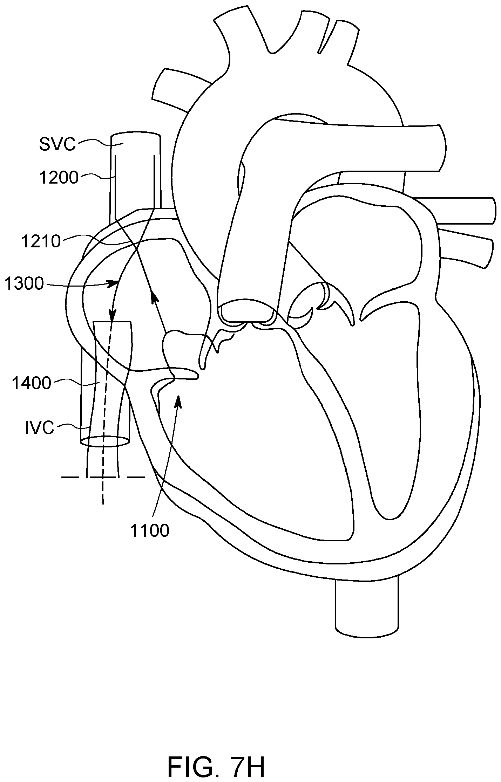

Referring again to Fig. 7G, as the catheter 1400 is withdrawn relative to the prosthetic heart valve 1100, the RVOT hook 1142 and posterior shelf 1144 may begin to hook backward and into contact with the ventricular or outflow side of the native tricuspid valve annulus. This contact is generally responsible for the prosthetic heart valve 1100 resisting migration into the right atrium. As the catheter 1400 is withdrawn farther relative to the prosthetic heart valve 1100, the remaining portions of the prosthetic heart valve 1100 (including the main body of the frame 1110 and the prosthetic leaflets 1120) may expand within the tricuspid valve annulus and begin to replace the functionality of the native tricuspid valve. Fig. 7H illustrates a further step in the procedure in which the catheter 1400 is further withdrawn and the prosthetic heart valve 1100 is allowed to fully expand into the native tricuspid valve. The catheter 1400 may be maneuvered so that the tether 1300, which is already fixed to the prosthetic heart valve 1100 and which extends through the catheter 1400 proximally, is looped around the arch 1210 of the anchor 1200. After the tether 1300 is looped around the arch 1210, the tether 1300 may be pulled proximally to tension the tether 1300, for example by manipulating the free end of the tether that is coupled to the delivery device or otherwise available for manipulation outside the patient's body. As the tether 1300 is being tensioned, the arch 1210 generally acts like a pulley, and tension on the tether 1300 may be increased pulling the RVOT hook 1142 and posterior shelf 1144 tighter against the outflow side of the native valve annulus. Once the tether 1300 is tensioned to the desired amount, it may then be affixed to the arch 1210 of the anchor 1200, for example via a knot, a separate accessory feature, or a barb-like feature built into the arch 1210. Once the tether 1300 is fixed to the arch 1210, the tension on the tether 1300 is effectively "locked," with the tether 1300 preventing the prosthetic heart valve 1100 from migrating into the right ventricle, and the RVOT hook 1142 and posterior shelf 1144 preventing the prosthetic heart valve 1100 from migrating into the right atrium. In other words, the prosthetic heart valve 1100 may be fully and satisfactorily anchored in the native tricuspid valve without any stent structure hooked around or otherwise contacting the inflow side of the native tricuspid valve, particularly in the area of the AV node.

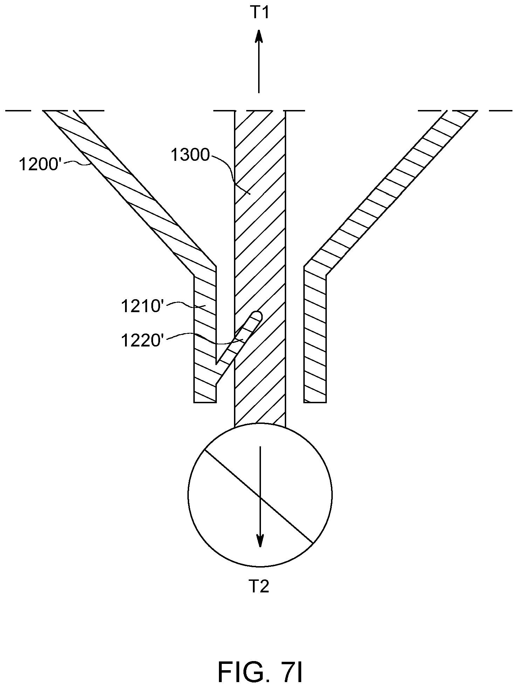

Fig. 7I is a schematic view of an alternate version of the anchor 1200' that includes a tether connection mechanism 1210' different than the arch 1210 of Fig. 7E. A portion of anchor 1200' is shown in Fig. 7I, with the anchor 1200' converging to a tether connection mechanism 1210' generally in the form of a cylinder that the tether 1300 passes through. A tine or barb 1220', which may be a piece of metal (*e.g.,* Nitinol) that is integral with the remainder of the anchor 1200', extends upwardly and inwardly from the tether connection mechanism 1210'. Preferably, the tine or barb 1220' has a sharp tip capable of digging into the tether 1300, for example if the tether 1300 is formed of a polymer. The tine 1220' is angled so that, if the tether 1300 is pulled in a first direction T1 aligned with the angle of the tine 1220', the tether 1300 can generally freely translate through the connection mechanism 1210', resulting in tension being added to the tether 1300. However, if the tether 1300 is pulled in the opposite direction T2, for example after releasing force on the tether 1300 while the tether is tensioned, the tine 1220' digs into the tether 1300, preventing the tether 1300 from translating any significant distance in the direction T2. Thus, with the tine 1220 angled so that the sharp tip is pointing superiorly, the tether 1300 may be pulled to the desired tension, and then upon reaching the desired tension, the tether 1300 may be released at which point the tine 1220' will engage the tether 1300 and lock the tether 1300 at the desired tension.

In some embodiments, after the tether 1300 has been tensioned to the desired amount and fixed to the anchor 1200 or 1200' at the desired tension, the remaining length of the tether 1300 extending beyond the anchor 1200 or 1200' may be cut and removed from the body, for example via a cautery tool introduced into the heart.

In some embodiments, it may be desirable for the prosthetic heart valve 1100 to be rotatable about a central longitudinal axis prior to or during deployment. For example, the RVOT hook 1142 is intended to be positioned at or near the RVOT, while the posterior shelf 1144 is intended to be positioned toward the ventricular wall opposite the interventricular septum. If the RVOT hook 1142 and posterior shelf 1144 are not in the desired rotational orientation prior to (or during) deployment, it may be desirable to have a mechanism to rotate the prosthetic heart valve 1100 to the desired rotational orientation relative to the native tricuspid valve. If the prosthetic heart valve 1100 is releasably coupled to an internal shaft or catheter during deployment, that internal shaft may be rotatable (*e.g.,* via manipulation of a handle of the delivery device) to re-orient the prosthetic heart valve 1100 into the desired rotational position.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A prosthetic heart valve (100) comprising:
a collapsible and expandable frame (200) that, in an expanded condition, includes a central portion (203), an atrial portion (202) flaring radially outwardly from the central portion (203), and a ventricular portion (204) flaring radially outwardly from the central portion (203);
a tube (300) positioned within the frame (200), the tube having a lumen extending along a longitudinal axis from the atrial portion (202) toward the ventricular portion (204) of the frame (200), wherein the tube (300) is formed of tissue or fabric;
a plurality of prosthetic leaflets (400) directly coupled to the tube (300) to form a valve, the valve allowing blood to flow through the lumen of the tube in an antegrade direction but substantially blocking blood from flowing through the lumen of the tube in a retrograde direction; and
a plurality of cords (600) each having a first portion coupled to the frame (200) and a second portion coupled to the tube (300), each of the plurality of cords (600) extending in a radial direction toward the longitudinal axis,
wherein the tube (300) excludes any metal structure directly attached to the tube (300).

2. The prosthetic heart valve of any of the preceding claims, wherein each of the plurality of cords (600) is a suture.

3. The prosthetic heart valve (100) of any of the preceding claims, further comprising a skirt (500) coupled to the frame (200), the skirt (500) including an atrial portion extending radially inwardly from the frame (200) and being connected to a first end of the tube (300), and a ventricular portion (520) extending radially inwardly from the frame (200) and being connected to a second end of the tube (300).

4. The prosthetic heart valve (100) of claim 3, wherein the skirt (500) is formed of a woven synthetic fabric.

5. The prosthetic heart valve (100) of claim 4, wherein the skirt (500) is formed of PET, PTFE, or UHMWPE.

6. The prosthetic heart valve (100) of any of claims 3-5, wherein the skirt (500) includes an outer section (510) attached to the frame (200), the outer section (510) being configured to contact a native valve annulus upon implantation to help seal against the native valve annulus and to prevent paravalvular leakage.

7. The prosthetic heart valve (100) of any of claims 3-6, wherein the ventricular portion (520) of the skirt (500) is substantially planar.

8. The prosthetic heart valve (100) of any of claims 3-7, wherein the ventricular portion (520) of the skirt (500) has an annular shape.

9. The prosthetic heart valve (100) of any of claims 3-8, wherein the atrial portion of the skirt (500) is substantially planar.

10. The prosthetic heart valve (100) of any of claims 3-9, wherein the atrial portion of the skirt (500) has an annular shape.

11. The prosthetic heart valve (100) of any of the preceding claims, wherein the plurality of cords (600) include a first group of cords (600) provided at an outflow end of the prosthetic heart valve (100) and a second group of cords (600) provided at an inflow end of the prosthetic heart valve (100).

12. The prosthetic heart valve (100) of any of the preceding claims, wherein the plurality of cords (600) only provide support in tension, helping to minimize movement of the tube (300) and the plurality of prosthetic leaflets (400) directly coupled to the tube (300) during normal operation of the prosthetic heart valve (100).

13. The prosthetic heart valve (100) of any of the preceding claims, wherein upon compression of the frame (200), compressive force is not translated via the plurality of cords (600) to the tube (300).

## Patentansprüche

1. Eine Herzklappenprothese (100), die Folgendes aufweist:
einen kollabierbaren und expandierbaren Rahmen (200), der in einem expandierten Zustand einen zentralen Abschnitt (203), einen atrialen Abschnitt (202), der sich von dem zentralen Abschnitt (203) radial nach außen erweitert, und einen ventrikulären Abschnitt (204) aufweist, der sich von dem zentralen Abschnitt (203) radial nach außen erweitert;
einen Schlauch (300), der innerhalb des Rahmens (200) positioniert ist, wobei der Schlauch ein Lumen aufweist, das sich entlang einer Längsachse von dem atrialen Abschnitt (202) zu dem ventrikulären Abschnitt (204) des Rahmens (200) erstreckt, wobei der Schlauch (300) aus Gewebe oder Stoff gebildet ist;
eine Mehrzahl von Prothesen-Klappensegeln (400), die direkt mit dem Schlauch (300) verbunden sind, um ein Ventil zu bilden, wobei das Ventil den Blutfluss durch das Lumen des Schlauchs in einer antegraden Richtung ermöglicht, aber im Wesentlichen den Blutfluss durch das Lumen des Schlauchs in einer retrograden Richtung blockiert; und
eine Mehrzahl von Fäden (600), von denen jeder einen ersten Abschnitt aufweist, der mit dem Rahmen (200) verbunden ist, und einen zweiten Abschnitt, der mit dem Schlauch (300) verbunden ist, wobei sich jeder der Mehrzahl von Fäden (600) in einer radialen Richtung zur Längsachse hin erstreckt,
wobei der Schlauch (300) keine direkt am Schlauch befestigte Metallstruktur aufweist (300).

2. Die Herzklappenprothese nach Anspruch 1, wobei jeder der mehreren Fäden (600) eine Naht bildet.

3. Die Herzklappenprothese (100) nach einem der vorhergehenden Ansprüche, die ferner eine mit dem Rahmen (200) verbundene Schürze (500) aufweist, wobei die Schürze (500) einen atrialen Abschnitt aufweist, der sich von dem Rahmen (200) radial nach innen erstreckt und mit einem ersten Ende des Schlauchs (300) verbunden ist, und einen ventrikulären Abschnitt (520), der sich von dem Rahmen (200) radial nach innen erstreckt und mit einem zweiten Ende des Schlauchs (300) verbunden ist.

4. Die Herzklappenprothese (100) nach Anspruch 3, wobei die Schürze (500) aus einem gewebten synthetischen Stoff gebildet ist.

5. Die Herzklappenprothese (100) nach Anspruch 4, wobei die Schürze (500) aus PET, PTFE oder UHMWPE gebildet ist.

6. Die Herzklappenprothese (100) nach einem der Ansprüche 3 bis 5, wobei die Schürze (500) einen äußeren Abschnitt (510) aufweist, der an dem Rahmen (200) befestigt ist, wobei der äußere Abschnitt (510) so konfiguriert ist, dass er bei der Implantation einen nativen Klappenring berührt, um die Abdichtung gegen den nativen Klappenring zu unterstützen und eine paravalvuläre Leckage zu verhindern.

7. Die Herzklappenprothese (100) nach einem der Ansprüche 3-6, wobei der ventrikuläre Abschnitt (520) der Schürze (500) im Wesentlichen planar ist.

8. Die Herzklappenprothese (100) nach einem der Ansprüche 3-7, wobei der ventrikuläre Abschnitt (520) der Schürze (500) eine ringförmige Form aufweist.

9. Die Herzklappenprothese (100) nach einem der Ansprüche 3 bis 8, wobei der atriale Abschnitt der Schürze (500) im Wesentlichen planar ist.

10. Die Herzklappenprothese (100) nach einem der Ansprüche 3 bis 9, wobei der atriale Abschnitt der Schürze (500) eine ringförmige Form aufweist.

11. Die Herzklappenprothese (100) nach einem der vorangehenden Ansprüche, wobei die Mehrzahl von Fäden (600) eine erste Gruppe von Fäden (600) aufweist, die an einem Ausflussende der Herzklappenprothese (100) vorgesehen ist, und eine zweite Gruppe von Fäden (600), die an einem Einflussende der Herzklappenprothese (100) vorgesehen ist.

12. Die Herzklappenprothese (100) nach einem der vorangehenden Ansprüche, wobei die Fäden (600) nur unter Spannung stützen und dazu beitragen, die Bewegung des Schlauches (300) und der Mehrzahl von Prothesen-Klappensegeln (400) zu minimieren, die während des normalen Betriebs der Herzklappenprothese (100) direkt mit dem Schlauch (300) verbunden sind.

13. Die Herzklappenprothese (100) nach einem der vorhergehenden Ansprüche, wobei beim Zusammendrücken des Rahmens (200) die Druckkraft nicht über die Mehrzahl von Fäden (600) auf den Schlauch (300) übertragen wird.

## Revendications

1. En valve cardiaque prothétique (100) comprenant :
un cadre pliable et extensible (200) qui, dans un état d'expansion, comprend une partie centrale (203), une partie auriculaire (202) s'évasant radialement vers l'extérieur à partir de la partie centrale (203), et une partie ventriculaire (204) s'évasant radialement vers l'extérieur à partir de la partie centrale (203) ;
un tube (300) placé à l'intérieur du cadre (200), le tube ayant une lumière qui s'étend le long d'un axe longitudinal de la partie auriculaire (202) vers la partie ventriculaire (204) du cadre (200), le tube (300) étant formé d'un tissu ou d'une étoffe ;
une pluralité de feuillets prothétiques (400) directement couplés au tube (300) pour former une valve, la valve permettant au sang de s'écouler à travers la lumière du tube dans une direction antérograde mais empêchant substantiellement le sang de s'écouler à travers la lumière du tube dans une direction rétrograde ; et
une pluralité de cordes (600) ayant chacune une première partie couplée au cadre (200) et une seconde partie couplée au tube (300), chacune de pluralité de cordes (600) s'étendant dans une direction radiale vers l'axe longitudinal,
dans lequel le tube (300) exclut structure métallique directement attachée au tube (300).

2. La valve cardiaque prothétique de la revendication 1, dans laquelle chacun des cordons (600) est une suture.

3. La valve cardiaque prothétique (100) de l'une quelconque des revendications précédentes, comprenant en outre une jupe (500) couplée au cadre (200), la jupe (500) comprenant une partie auriculaire s'étendant radialement vers l'intérieur du cadre (200) et étant reliée à une première extrémité du tube (300), et une partie ventriculaire (520) s'étendant radialement vers l'intérieur du cadre (200) et étant reliée à une seconde extrémité du tube (300).

4. Valve cardiaque prothétique (100) de la revendication 3, dans laquelle la jupe (500) est formée d'un tissu synthétique tissé.

5. La valve cardiaque prothétique (100) de la revendication 4, dans laquelle la jupe (500) est formée de PET, PTFE ou UHMWPE.

6. La valve cardiaque prothétique (100) de l'une des revendications 3 à 5, dans laquelle la jupe (500) comprend une partie externe (510) fixée au cadre (200), la partie externe (510) étant configurée pour entrer en contact avec un anneau valvulaire natif lors de l'implantation afin de contribuer à l'étanchéité contre l'anneau valvulaire natif et d'empêcher les fuites paravalvulaires.

7. La valve cardiaque prothétique (100) de l'une des revendications 3 à 6, dans laquelle la partie ventriculaire (520) de la jupe (500) est sensiblement plane.

8. La valve cardiaque prothétique (100) de l'une des revendications 3 à 7, dans laquelle la partie ventriculaire (520) de jupe (500) a une forme annulaire.

9. La valve cardiaque prothétique (100) de l'une des revendications 3 à 8, dans laquelle la partie auriculaire de la jupe (500) est sensiblement plane.

10. La valve cardiaque prothétique (100) de l'une des revendications 3 à 9, dans laquelle la partie auriculaire de la jupe (500) a une forme annulaire.

11. La valve cardiaque prothétique (100) de l'une des revendications précédentes, dans laquelle la pluralité de cordons (600) comprend un premier groupe de cordons (600) fourni une extrémité de sortie de la valve cardiaque prothétique (100) et un second groupe de cordons (600) fourni à une extrémité d'entrée de la valve cardiaque prothétique (100).

12. La valve cardiaque prothétique (100) de l'une quelconque des revendications précédentes, dans laquelle la pluralité cordons (600) ne fournit un soutien qu'en tension, contribuant à minimiser le mouvement du tube (300) et la pluralité de feuillets prothétiques (400) directement couplés au tube (300) pendant le fonctionnement normal de la valve cardiaque prothétique (100).

13. La valve cardiaque prothétique (100) de l'une des revendications précédentes, dans laquelle, lors de la compression du cadre (200), la force de compression n'est pas transmise au tube (300) par l'intermédiaire de la pluralité de cordons (600).
